(19) Europäisches Patentamt
European Patent Office
Office européen des brevets

(11) EP 2 143 419 A2

(12) **EUROPÄISCHE PATENTANMELDUNG**

(43) Veröffentlichungstag:
**13.01.2010 Patentblatt 2010/02**

(21) Anmeldenummer: **09156013.6**

(22) Anmeldetag: **24.03.2009**

(51) Int Cl.:
***A61K 8/73*** *(2006.01)* ***A61K 8/86*** *(2006.01)*
***A61Q 5/10*** *(2006.01)*

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(30) Priorität: **09.07.2008 DE 102008032178**

(71) Anmelder: **Henkel AG & Co. KGaA**
**40589 Düsseldorf (DE)**

(72) Erfinder:
- **Goutsis, Konstantin**
**41812, Erkelenz (DE)**
- **Reichert, Anja**
**40629, Düsseldorf (DE)**

(54) **Oxidationsfärbemittel mit Gelbasis aus hochethoxylierten Fettalkoholen und polysaccharidischem Verdicker für pH-reduzierte Expressfärbung**

(57) Gegenstand der Erfindung ist ein Mittel zum Färben von keratinischen Fasern, das durch Vermischung von Zubereitungen aus mindestens zwei getrennt konfektionierten Containern hergestellt wird, wobei ein Container ein gelförmiges Färbemittel (a), enthaltend in einem kosmetischen Träger mindestens ein Oxidationsfarbstoffvorprodukt, und ein weiterer Container eine Oxidationsmittelzubereitung (b), enthaltend mindestens ein Oxidationsmittel, enthält. Das ein gelförmiges Färbemittel (a) ist dadurch gekennzeichnet, dass die Gelbasis des Färbemittel (a) eine nichtionische Emulgatorkombination aus mindestens zwei ethoxylierten, linearen Fettalkoholen mit unterschiedlichem Ethoxylierungsgrad und mindestens einen polymeren Verdicker enthält, das anwendungsbereite Mittel einen pH-Wert von 7 bis 8,9 besitzt, und das anwendungsbereite Mittel anionische Tenside in einem Gesamtgehalt von weniger als 0,3 Gew.-%, bezogen auf das Gesamtgewicht der Anwendungszubereitung, enthält. Die erfindungsgemäßen Mittel zeichnen sich durch gute Färbeeigenschaften bei gleichzeitigem Schutz vor Schädigungen für das menschliche Haar aus.

EP 2 143 419 A2

**Beschreibung**

Die Erfindung betrifft ein oxidatives Färbemittel für keratinhaltige Fasern für pH-reduzierte und damit haarschonende Färbungen. Dieses Färbemittel enthält in einem wässrigen Träger mindestens ein Oxidationsfarbstoffvorprodukt sowie eine Färbecremebasis, welche aus ethoxylierten Fettalkoholen mit unterschiedlichem Ethoxylierungsgrad sowie einem verdickenden, polysaccharidischen Polymer gebildet wird und welche gleichzeitig frei von anionischen Tensiden ist. Weiterhin betrifft die Erfindung ein Verfahren mit verkürzter Einwirkzeit dieser oxidativen Färbemittel auf keratinhaltigen Fasern. Darüber hinaus betrifft die Erfindung die Verwendung des besagten Mittels zur Färbung von Haaren und zur haarschonenden Grauabdeckung.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus.

Eine weitere Möglichkeit zur Farbveränderung bietet die Verwendung von Färbemitteln, welche sogenannte Oxofarbstoffvorprodukte enthalten. Oxofarbstoffvorprodukte sind im Allgemeinen selbst keine Farbstoffe, und eignen sich daher jede für sich genommen allein nicht zur Färbung keratinhaltiger Fasern. In Kombination bilden sie in einem nichtoxidativen Prozess der sogenannten Oxofärbung Farbstoffe aus. Die resultierenden Färbungen besitzen teilweise Farbechtheiten auf der keratinhaltigen Faser, die mit denen der Oxidationsfärbung vergleichbar sind.

Sollen Substrate aufgehellt oder gar gebleicht werden, werden die das Substrat färbenden Farbstoffe meist oxidativ unter Einsatz von entsprechenden Oxidationsmitteln, wie beispielsweise Wasserstoffperoxid, entfärbt.

Insbesondere oxidative Haarfärbemittel sind trotz ihrer vorteilhaften Färbeeigenschaften für den Anwender mit Nachteilen behaftet.

Erstens führt der Einsatz der Oxidationsmittel zur Ausfärbung beziehungsweise Entwicklung der eigentlichen Färbung zu Schädigungen in der Haarstruktur und auf der Haaroberfläche. Das Haar wird brüchig, seine Elastizität lässt nach und die Kämmbarkeit nimmt ab. Diese Schädigung nimmt mit der Anwendungsdauer zu. Handelsübliche oxidative Färbemittel müssen meist über einen Zeitraum von 30 Minuten und länger auf die Haarfaser einwirken. Sollen graue oder weiße Haare kaschiert werden, so muss für eine bessere Grauabdeckung die Einwirkzeit eines oxidativen Färbemittels gegebenenfalls noch weiter erhöht werden. Die Erhöhung der Einwirkzeit führt zu einer gesteigerten Beeinträchtigung der Haarstruktur. Es ist auch aus Gründen des Anwendungskomforts nahe liegend, dass bei den Benutzern solcher Haarfärbemittel ein Bedürfnis besteht, diese Einwirkungszeit zu verringern.

Zweitens benötigen oxidative Färbemittel in der Regel einen basischen pH-Wert zur Ausfärbung, insbesondere zwischen pH 9,0 und pH 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Das basische Milieu stellt jedoch einen weiteren Grund der Schädigung für das Haar und dessen Struktur dar, der ebenfalls mit gesteigerter Anwendungszeit an Bedeutung gewinnt. Die Spreizung der äußeren Schuppenschicht führt außerdem zu einem unangenehmen Oberflächenempfinden der Haare und damit zu einer verschlechterten Kämmbarkeit im Nass- und Trockenzustand. Dadurch besteht für den Verbraucher eine gesteigerte Notwendigkeit,

zusätzliche Nachbehandlungsmittel wie Konditioniermittel einsetzen. Solche Konditioniermittel sind üblicherweise insbesondere notwendig bei Verwendung von Mitteln mit einem hohen Anteil an anionischem Tensid, um der Austrocknung von Haar- und Hautoberfläche entgegenzuwirken.

Schließlich wird der basische pH-Wert häufig mit Ammoniak als Alkalisierungsmittel eingestellt, da ammoniakhaltige Färbemittel zusätzliche Vorteile hinsichtlich der Färbeleistung besitzen. Für den Anwender besitzt ein solches Färbemittel jedoch den Nachteil, dass es durch Ammoniak neben zusätzlichen Schädigungen des Haares zu Reizungen von Augen oder Kopfhaut kommen kann, wodurch Sensibilisierungen oder gar allergische Reaktionen hervorgerufen werden können. Darüber hinaus besitzen solche Färbemittel einen intensiven, unangenehmen Geruch, der schlimmstenfalls auch zu Reizungen der Nasenschleimhaut führen kann. Ausserdem kann auch die Produktion und Lagerung von Ammoniak-haltigen Färbemitteln mit Problemen hinsichtlich Handhabbarkeit und Stabilität verbunden sein.

Aufgabe der vorliegenden Erfindung ist es daher, die oben genannten Nachteile oxidativer Haarfärbemittel herabzusenken. Die Färbemittel sollen das Haar schützen und damit eine verringerte Schädigung des Haares bewirken. Insbesondere Schutz vor oxidativen Schädigungen der Haarstruktur und der Haaroberfläche soll durch die Haarfärbemittel erzielt werden. Besonders wünschenswert sind pflegende Eigenschaften der Mittel, so dass der Anwender auf den Einsatz zusätzlicher Konditioniermittel verzichten kann. Vorzugsweise sollte das Mittel dazu auf anionische Tenside weitestgehend oder vollständig verzichten, um der zusätzlichen Austrocknung von Haar- und Hautoberfläche entgegenzuwirken. Die Färbemittel sollen außerdem ein Verdickungssystem enthalten, welches einen hohen Gehalt an Farbstoffvorprodukten zulässt, und gleichzeitig über eine ausreichende Lagerstabilität verfügen. Der pH-Wert der Anwendungsmischung soll zur Verringerung von Haarschädigungen möglichst schwach alkalisch sein. Die Färbemittel sollen schließlich eine Verkürzung der Anwendungsdauer zulassen, wodurch eine weitere Reduktion der Schädigung ermöglicht wird. Die Verringerung von Haarschädigungen während der Färbung soll jedoch nicht zu Lasten einer verringerten Färbeleistung der Mittel erreicht werden. Insbesondere soll das Färbemittel eine verbesserte Grauabdeckung gegenüber herkömmlichen Färbemitteln bei einer Reduktion des pH-Wert erzielen. Gleichsam ist die Verkürzung der Anwendungszeit für den Verbraucher als Erleichterung in der Anwendung erstrebenswert. Schließlich ist es wünschenswert, oxidative Haarfärbemittel bereitzustellen, die weitestgehend oder vollständig auf den Zusatz von Ammoniak verzichten.

In nicht vorhersehbarer Weise wurde nun gefunden, dass eine neuartige Färbecremegrundlage auf Basis einer Kombination von hochethoxylierten Fettalkohol-Emulgatoren mit unterschiedlich hohen Ethoxylierungsgraden und einem polysaccharidischen Verdickungsmittel die Bereitstellung von Färbemitteln mit einem hohen Gehalt an Oxidationsfarbstoffvorprodukten ermöglicht. Die erfindungsgemäßen Färbemittel verzichten weiterhin auf anionische Tenside, wodurch einerseits die Stabilität der Färbecreme verbessert wird und andererseits übliche Nachteile von anionischen Tensiden in der Anwendung verringert werden. Bedingt durch die Absenkung des pH-Werts bei Anwendung des erfindungsgemäßen Mittels kann die Haarschädigung signifikant minimiert werden. Die Absenkung des pH-Werts in dem Mitteln wird dabei ohne Einschränkungen im Grauabdeckungsvermögen erreicht. Auch eine Verkürzung der Einwirkzeit unter Erzielung eines dem Stand der Technik entsprechenden Färbeeffekts ist auf diese Weise möglich.

Ein erster Gegenstand der Erfindung ist daher ein Mittel zum Färben keratinischer Fasern, insbesondere menschlicher Haare, bestehend aus mindestens zwei getrennt konfektionierten Containern, wobei ein Container ein gelförmiges Färbemittel (a), enthaltend in einem kosmetischen Träger mindestens ein Oxidationsfarbstoffvorprodukt, und ein weiterer Container eine Oxidationsmittelzubereitung (b), enthaltend mindestens ein Oxidationsmittel, enthält, **dadurch gekennzeichnet, dass**

i. die Gelbasis des Färbemittel (a)

  0,5 bis 10 Gew.-%, bezogen auf das Gesamtgewicht des anwendungsbereiten Mittels, einer nichtionischen Emulgatorkombination aus mindestens zwei ethoxylierten, linearen Fettalkoholen mit 8 bis 22 Kohlenstoffatomen,
  wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 40 bis 100 Mol Ethylenoxid besitzt,
  und mindestens einen polymeren Verdicker enthält,

ii. das anwendungsbereite Mittel einen pH-Wert von 7 bis 8,9 besitzt, und
iii. das anwendungsbereite Mittel anionische Tenside in einem Gesamtgehalt von weniger als 0,3 Gew.-%, bezogen auf das Gesamtgewicht der Anwendungszubereitung, enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische

Träger ist im Sinne der Erfindung ist wässrig, alkoholisch oder wässrig-alkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel.

Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als ersten wesentlichen Inhaltsstoff enthält das erfindungsgemäße Mittel ein Färbemittel, welches auf einer Gelbasis beruht, die durch eine spezifische Kombination von mindestens zwei nichtionischen Emulgatoren und mindestens einem verdickenden, polysaccharidischen Polymer beruht.

Die nichtionischen Emulgatoren sind dabei ausgewählt aus ethoxylierten, linearen Fettalkoholen, bevorzugt einer Kettenlänge mit 8 bis 22 Kohlenstoffatomen. Unter einem ethoxylierten Fettalkohol wird im Sinne der Erfindung ein Anlagerungsprodukt von Ethylenoxid an einen Fettalkohol verstanden, wobei der Ethoxylierungsgrad die Molmenge Ethylenoxid (EO) angibt, die durchschnittlich pro Mol Fettalkohol angelagert wurde. Es ist überraschend gefunden worden, dass die Kombination aus mindestens einem ethoxylierten Fettalkohol mit einen mittleren, durchschnittlichen Ethoxylierungsgrad und mindestens einem ethoxylierten Fettalkohol mit einen hohen, durchschnittlichen Ethoxylierungsgrad eine hohe Konzentration an Oxidationsfarbstoffvorprodukten in den Gelen gestattet und einer hervorragenden Lagerstabilität der Gele gewährleistet.

Bevorzugte ethoxylierte Fettalkohole sind Ethylenoxid-Anlagerungsprodukte an Caprinalkohol, Laurylalkohol, Isotridecylalkohol, Myristylalkohol, Cetylalkohol, Palmoleylalkohol, Stearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Besonders bevorzugt sind Anlagerungsprodukte an technische Fettalkohole bzw. deren Mischungen mit 12 bis 18 Kohlenstoffatomen wie beispielsweise Kokos-, Palm-, Palmkern- oder Talgfettalkohol, insbesondere Kokos- und/oder Talgfettalkohol.

Unter ethoxylierten Fettalkohol mit einem mittleren, durchschnittlichen Ethoxylierungsgrad wird im Sinne der Erfindung ein Anlagerungsprodukt von 15 bis 35 Mol Ethylenoxid pro Mol Fettalkohol verstanden.

Ein ethoxylierter Fettalkohol mit einem hohen, durchschnittlichen Ethoxylierungsgrad ist im Sinne der Erfindung ein Anlagerungsprodukt von 40 bis 100 Mol Ethylenoxid pro Mol Fettalkohol verstanden. Bevorzugte Fettalkohole mit einem hohen, durchschnittlichen Ethoxylierungsgrad besitzen einen Ethoxylierungsgrad von 40 bis 60, und ganz besonders bevorzugt von 45 bis 55.

Je nach Herstellungsmethode fallen die erfindungsgemäßen ethoxylierten Fettalkohole als ein Gemisch mit einer unterschiedlichen Ethoxylierungsgradverteilung an. Im Sinne der Erfindung werden diese Emulgatoren daher nach dem durchschnittlichen Ethoxylierungsgrad gekennzeichnet. Dieser ist üblicherweise als Ziffer hinter dem Fettalkohol-Suffix "eth-" in der INCI-Bezeichnung erkennbar.

Bevorzugte ethoxylierte Fettalkohole mit einem mittleren, durchschnittlichen Ethoxylierungsgrad sind beispielsweise die Handelsprodukte: Ceteth-15 (Nikkol® BC 15 TX, Firma Nikko Chemicals Co., Ltd.), Laneth-15 (Polychol® 15, Firma Croda), Ceteareth-15 (Eumulgin® CS 15, Firma Cognis); Laneth-16 (und) Ceteth-16 (und) Oleth-16 (und) Steareth-16 (als Gemisch vertrieben unter dem Handelsnamen Solulan® 16, Firma Noveon); Oleth-20 (Ritoleth® 20, Firma Rita Corp.), Ceteth-20 (Brij® 58 SP, Firma Uniqema; Lipocol® C 20, Firma Lipo Chemicals Inc.), Ceteareth-20 (Surfac® JH 200, Firma Surfachem; Eumulgin® B 2, Firma Cognis), Laneth-20 (Polychol® 20, Firma Croda); Steareth-21 (Brij® 721 P, Firma Uniqema; Eumulgin® S 21, Firma Cognis); Ceteareth-23 (Mergital® C 23, Firma Cognis), Laureth-23 (Canasol® BJ 35, Firma Canamax); Ceteareth-25 (Cremophor® A 25, Firma BASF); Ceteareth-27 (Plurafac® A 38, Firma BASF); Ceteareth-30 (Lipocol® SC 30, Firma Lipo Chemicals; Eumulgin® B 3, Firma Cognis).

Bevorzugte ethoxylierte Fettalkohole mit einem hohen, durchschnittlichen Ethoxylierungsgrad sind beispielsweise die Handelsprodukte: Ceteth-40 (Nikkol® BC 40 TX, Firma Nikko Chemicals Co., Ltd.), Laneth-40 (Polychol® 40, Firma Croda); Oeth-50 (Nikkol® BO 50 V, Firma Nikko Chemicals Co., Ltd.), Ceteareth-50 (Genapol® T 500, Firma Clariant; Mergital® CS 50, Firma Cognis); Ceteareth-60 (Findet® 1618 A 72, Firma KAO Corp.); Ceteareth-80 (Lutensol® AT 80, Firma BASF). Besonders bevorzugt ist Ceteareth-50.

Ethoxylierte Fettalkohole mit mittlerem und hohem, durchschnittlichen Ethoxylierungsgrad sind im anwendungsbereiten Mittel insgesamt zwischen 0,05 und 15 Gew.-%, bevorzugt 0,1 und 10 Gew.-% enthalten.

Erfindungsgemäß besonders bevorzugte Mittel sind **dadurch gekennzeichnet, dass** das Gewichtsverhältnis

der Menge der ethoxylierten Fettalkohole mit einem mittleren, durchschnittlichen Ethoxylierungsgrad zu der Menge der ethoxylierten Fettalkohole mit einem hohen, durchschnittlichen Ethoxylierungsgrad im Färbemittel (a) einen Wert von 3:1 bis 1:5, bevorzugt von 2:1 bis 1:3, besitzt.

Ein weiterer, wesentlicher Inhaltsstoff der Gelbasis im Färbemittel (a) ist mindestens ein polymerer Verdicker. Dadurch lässt sich sie Rheologie der erfindungsgemäßen Mittel lässt sich in die vom Verbraucher gewünschten cremigfließfähigen Systeme überführen.

Erfindungsgemäß bevorzugte Mittel sind **dadurch gekennzeichnet, dass** der polymerer Verdicker im Färbemittel (a) zu 0,005 bis 1,0 Gew.-%, insbesondere zu 0,01 bis 0,5 Gew.-%, und ganz besonders zu 0,05 bis 0,3 Gew.-%, jeweils bezogen auf das Gesamtgewicht der Anwendungszubereitung, enthalten ist.

Bevorzugt handelt es sich bei dem polymeren Verdicker um einen hydrophilen Verdicker. Dem Fachmann sind solche Verdicker bekannt. Erfindungsgemäß einsetzbare, polymere Verdicker sind insbesondere synthetische Polymere, polysaccharidische Polymere, anorganische Verdicker sowie deren Mischungen.

Als erfindungsgemäß geeignete, synthetische, polymere Verdicker sind unter anderen zu nennen:

- Polyacrylsäurepolymere (Carbomere), insbesondere Poly(meth)acrylate, weiterhin gegebenenfalls verzweigte Homo- oder Co-Polymere der Acrylsäure, ihrer Salze oder ihrer Alkylester, die gegebenenfalls kationisch oder anionisch in der Alkylkette modifiziert sind,
- Polyacrylamidpolymere, insbesondere Homo- oder Co-Polymere von Acrylsäureamid und/oder Methacrylsäureamid, die gegebenenfalls kationisch oder anionisch modifiziert sind,
- Copolymere aus Acrylsäure und Acrylsäureamid.

Verdickende, polysaccharidische Polymere sind Verbindungen mit einem Polysaccharid-Gerüst, die geeignet sind, die Viskositäten der Zubereitung zu erhöhen. Hierzu zählen beispielsweise Algin, Alginate, Glucane wie Dextran, Pullulan, Curdlan, Cellulose, Laminarin, Amylose oder Lichenin, Traganth, Karaya-Gummi, Ghatti-Gummi, Agar, Carrageenan, Chitin, Chitosan, Gummi arabicum, Gellan, Carob Gummi, Galactonmannane wie Guar oder Johannisbrotkernmehl, Tamarindenkernmehl oder deren Derivate.

Als erfindungsgemäße, anorganische Verdicker sind beispielsweise geeignete Elektrolyte, wie Natriumchlorid oder Kaliumchlorid, Schichtsilicate (polymere, kristalline Natriumdisilicate) und Magnesium Aluminium Silicate oder Bentonite, besonders Smektite, wie Montmorillonit oder Hectorit, die gegebenenfalls auch geeignet modifiziert sein können, zu nennen.

Es hat sich gezeigt, dass insbesondere verdickende, polysaccharidische Polymere milde und lagerstabile Formulierungen ergeben. Erfindungsgemäß bevorzugte polymere Verdicker des Färbemittels (a) sind daher verdickende, polysaccharidische Polymere.

Besonders bevorzugte Polysaccharide sind dabei Polymere auf Cellulose-Basis. Hierbei können chemisch modifizierten Cellulosen, wie beispielsweise Acetyl-, Methyl- oder Ethylcellulosen, Hydroxyalkylcellulosen oder Carboxyalkylcellulosen eingesetzt werden. Besonders bevorzugte Cellulosederivate besitzen saccharidische Seitenketten, wie beispielsweise Xanthan.

Eine besondere Ausführungsform der vorliegenden Erfindung ist **dadurch gekennzeichnet, dass** das verdikkende, polysaccharidische Polymer des Färbemittels (a) Xanthan ist.

Xanthan ist ein mikrobielles, anionisches Polysaccharid, das von Xanthomonas campestris unter geeigneten Kulturbedingungen ausgeschieden wird. Es enthält D-Glucose, D-Mannose, D-Glucuronsäure, Acetat und Pyruvat im molaren Verhältnis 28:30:20:17:5,1-6,3. Die Hauptkette besteht aus β-1,4-gebundenen Glucose-Einheiten (Cellulose-Kette). An jedem 2. Glucose-Rest hängt eine 3-gliedrige Seitenkette.

Die Molmasse von Xanthan variiert von $2 \cdot 10^6$ bis $12 \cdot 10^6$ Dalton - je nach Herstellungsbedingungen und Stamm-Material. Xanthan nimmt in wässriger Lösung relativ starre, regelmäßige, helikale Strukturen an. Es können Einfachhelices wie auch Doppelhelices vorliegen. Xanthan löst sich gut in heißem und kaltem Wasser. Dabei bilden die Helices ein dreidimensionales Netzwerk, welches hohe Viskositäten verursacht. Xanthan ist stärker pseudoplastisch als andere bekannte Hydrokolloide und bildet Gele mit hoher Gefrier-Tau-Stabilität.

Schließlich enthalten die erfindungsgemäßen Mittel mindestens ein Oxidationsfarbstoffvorprodukt. Die erfindungsgemäßen Mittel zum Färben keratinischer Fasern sind demnach **dadurch gekennzeichnet, dass** sie mindestens ein Oxidationsfarbstoffvorprodukt vom Entwicklertyp und/oder Kupplertyp enthalten. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Oxidationsfarbstoffvorprodukte werden bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide

oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können. Nachfolgend werden erfindungsgemäße Entwicklerkomponenten eingehender vorgestellt.

Besonders bevorzugt sind p-Phenylendiaminderivate. Besonders bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diamino-propan-2-ol, N,N'-Bis-(2-hydroxyethyl)-N,N'-bis-(4'-aminophenyl)ethylendiamin, N,N'-Bis-(4'-aminophenyl)-tetramethylendiamin, N,N'-Bis-(2-hydroxyethyl)-N,N'-bis-(4'-aminophenyl)tetramethylendiamin, N,N'-Bis-(4-(methylamino)phenyl)-tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-di-aminophenyl)-1,4,7,10-tetraoxadecan sowie ihre physiologisch verträglichen Salze. Ganz besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)propan-2-ol, N,N'-Bis-(4-aminophenyl)-1,4-diazacyclo-heptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methylphenol, 4-Amino-3-fluorphenol, 2-Hydroxymethylamino-4-aminophenol, 4-Amino-3-hydroxy-methylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)-phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze. Ganz besonders bevorzugte Verbindungen sind p-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-di-aminopyrimidin und 2,5,6-Triaminopyrimidin. Bevorzugte Pyrazol-Derivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Di-amino-3-tert-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze. Bevorzugte Pyrazolopyrimidin-Derivate sind insbesondere die Derivate des Pyrazolo[1,5-a]-pyrimidin und deren tautomeren Formen, sofern ein tautomeres Gleichgewicht besteht.Unter den Pyrazolo[1,5-a]pyrimidinen sind Verbindungen bevorzugt, die ausgewählt aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a] pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo-[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxy-ethyl)-amino]ethanol, 2-[(7-Aminopyrazolo[1,5-a]-pyrimidin-3-yl)-(2-hydroxyethyl)amino] ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo-[1,5-a]pyrimidin sowie ihre physiologisch verträglichen Salze.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N, N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxylgruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt: m-Aminophenol und/oder dessen Derivate, m-Diaminobenzol und/oder dessen Derivate, o-Diaminobenzol und/oder dessen Derivate, o-Aminophenolderivate, Naphthalinderivate mit mindestens einer Hydroxygruppe, Di- beziehungsweise Trihydroxybenzol und/oder deren Derivate, Pyridinderivate, Pyrimidinderivate, Monohydroxyindol-Derivate und/oder Monoaminoindol-Derivate, Monohydroxyindolin-Derivate und/oder Monoaminoindolin-Derivate, Pyrazolonderivate, Morpholinderivate, Chinoxalinderivate sowie Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung aus der Gruppe, die gebildet wird aus m-Phenylendiamin, 2-(2,4-Diaminophenoxy) ethanol, 1,3-Bis-(2,4-diaminophenoxy)propan, 1-Methoxy-2-amino-4-(2'-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)-ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino) benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet. Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Aus Stabilitätsgründen und unter Pflegeaspekten ist es bevorzugt, wenn die Mittel nur einen geringen Anteil an anionischen Tensiden enthalten. Efindungsgemäß bevorzugte Mittel sind daher weitestgehend frei von anionischen Tensiden. Weitestgehend frei von anionischen Tensiden im Sinne der Erfindung bedeutet, dass das anwendungsbereite Mittel anionische Tenside in einem Gesamtgehalt von weniger als 0,3 Gew.-%, bezogen auf das Gesamtgewicht der Anwendungszubereitung, enthält. esonders bevorzugte Mittel sind **dadurch gekennzeichnet, dass** das Färbemittel (a) frei von anionischen Tensiden ist.

Als anionische Tenside verstehen sich im Sinne der Erfindung alle für die Verwendung am menschlichen Körper geeigneten anionischen oberflächenaktiven Stoffe. Diese sind gekennzeichnet durch eine wasserlöslich machende, anionische Gruppe wie beispielsweise eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen); Ethercarbonsäuren der Formel $RO(CH_2CH_2O)_xCH_2COOH$, in der R eine lineare Alkylgruppe mit 8 bis 30 C-Atomen und x = 0 oder einer Zahl von 1 bis 16 ist; Acylsarcoside mit 8 bis 24 C-Atomen in der Acylgruppe; Acyltauride mit 8 bis 24 C-Atomen in der Acylgruppe; Acylisethionate mit 8 bis 24 C-Atomen in der Acylgruppe; Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen; lineare Alkansulfonate mit 8 bis 24 C-Atomen; lineare $\alpha$-Olefinsulfonate mit 8 bis 24

C-Atomen; Sulfonate ungesättigter Fettsäuren mit 8 bis 24 C-Atomen und 1 bis 6 Doppelbindungen; α-Sulfofettsäuremethylester von Fettsäuren mit 8 bis 30 C-Atomen; Alkylsulfate und Alkylethersulfate der Formel $RO(CH_2CH_2O)_xSO_3H$, in der R eine bevorzugt lineare Alkylgruppe mit 8 bis 30 C-Atomen und x = 0 oder 1 bis 12 ist; Gemische oberflächenaktiver Hydroxysulfonate, sulfatierte Hydroxyalkylpolyethylen- und/oder Hydroxyalkylenpropylenglykolether; Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen; Alkyl- und/oder Alkenyletherphosphate; sulfatierte Fettsäurealkylenglykolester; Monoglyceridsulfate und Monoglyceridethersulfate.

Insbesondere enthalten die erfindungsgemäßen Färbemittel (a) weniger als 0,3 Gew.-%, bezogen auf das Gesamtgewicht der anwendungsbereiten Mittel, an anionischen Tensiden, ausgewählt aus Alkylsulfaten, Alkylethersulfaten und Ethercarbonsäuren mit 10 bis 18 C-Atomen in der Alkylgruppe und bis zu 12 Glykolethergruppen im Molekül, und ganz besonders bevorzugt keine anionische Tenside.

Um die Keratinfasern und auch die Haut so weit wie möglich zu schonen, ist die Einstellung eines zu hohen pH-Wertes nicht wünschenswert. Ein weiterer wesentlicher Aspekt der erfindungsgemäßen Mittel ist daher, dass das gebrauchsfertige Haarfärbepräparat einen pH-Wert im Bereich von 7 bis 8,9 aufweist. Besonders bevorzugt ist die Anwendung der Haarfärbemittel in einem schwach alkalischen Milieu, bevorzugt bei einem pH-Wert im Bereich von 7,5 bis 8,5. Zur Einstellung des pH-Werts sind dem Fachmann gängige Acidifizierungs- und Alkaliserungsmittel geläufig. Die zur Einstellung des bevorzugten pH-Wertes erfindungsgemäß verwendbaren Alkalisierungsmittel können aus der Gruppe, die gebildet wird aus basischen Aminosäuren, Aminen, Ammoniak, Alkalimetallhydroxiden, Alkalimetallmetasilikaten, Alkalimetallphosphaten und Alkalimetallhydrogenphosphaten ausgewählt werden. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Bei den pH-Werten im Sinne der vorliegenden Erfindung handelt es sich um pH-Werte, die bei einer Temperatur von 25 °C gemessen wurden.

Eine weiterhin bevorzugte Ausführungsform des erfindungsgemäßen Mittels ist **dadurch gekennzeichnet, dass** das Färbemittel (a) eine Kombination von mindestens zwei Alkalisierungsmitteln enthält, wobei mindestens ein Alkalisierungsmittel ein Alkanolamin ist, ausgewählt aus der Gruppe, die gebildet wird aus Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol, 2-Aminobutanol, N,N-Dimethyl-ethanolamin, Methylglucamin, Triethanolamin, Diethanolamin und Triisopropanolamin, und wobei mindestens ein Alkalisierungsmittel eine basische Aminosäure ist. Das Alkanolamin ist in den erfindungsgemäßen Oxidationsfärbemitteln bevorzugt in Mengen von 0,5 bis 15 Gew.-%, insbesondere von 2 bis 5 Gew.-%, bezogen auf das Gesamtgewicht der Anwendungsmischung, enthalten. Besonders bevorzugt sind dabei Mittel, die als Alkanolamin Monoethanolamin enthalten.

Als basische Aminosäure im Sinne der Erfindung gilt eine organische Verbindung, die in ihrer Struktur mindestens eine protonierbare Aminogruppe und mindestens eine -COOH- oder eine $-SO_3H$-Gruppe aufweist, und deren isoelektrischer Punkt (pl) einen Wert von 7,0 oder größer, gemessen unter Standardbedingungen, besitzt. Bevorzugte basische Aminosäuren sind Aminocarbonsäuren, insbesondere α-Aminocarbonsäuren und ω-Aminocarbonsäuren. Unter den α-Aminocarbonsäuren sind wiederum Lysin, Ornithin, Guanidin und Arginin besonders bevorzugt. Die basischen Aminosäuren können den erfindungsgemäßen Mitteln bevorzugt in freier Form zugegeben werden. In einer Reihe von Fällen ist es jedoch auch vorteilhaft, die Aminosäuren in Salzform einzusetzen. Bevorzugte Salze sind dann die Verbindungen mit Halogenwasserstoffsäuren oder Schwefelsäure, insbesondere die Hydrochloride, die Hydrobromide und die Sulfate. Weiterhin können die basischen Aminosäuren auch in Form von Oligopeptiden und Proteinhydrolysaten eingesetzt werden, wenn sichergestellt ist, dass die erforderlichen Mengen der erfindungsgemäß eingesetzten Aminosäuren darin enthalten sind. Eine erfindungsgemäß besonders bevorzugte basische Aminosäure ist Arginin, insbesondere in freier Form, aber auch als Hydrochlorid eingesetzt. Die basische Aminosäure ist in den erfindungsgemäßen Oxidationsfärbemitteln bevorzugt in Mengen von 0,01 bis 10 Gew.-%, insbesondere von 0,05 bis 5 Gew.-%, bezogen auf das Gesamtgewicht der Anwendungsmischung, enthalten.

Als Alkalisierungsmittel ist ebenso Ammoniak in den erfindungsgemäßen Färbemitteln einsetzbar. Insbesondere Färbeergebnisse lassen sich durch den Einsatz von Ammoniak anstelle anderer Alkalisierungsmittel häufig positiv beeinflussen. Andererseits ist die Verwendung von Ammoniak aus den weiter oben ausgeführten Gründen nicht immer wünschenswert. Es ist daher erfindungsgemäß besonders vorteilhaft, wenn das Mittel ammoniakarm, bevorzugt ammoniakfrei ist.

Die Begriffe "ammoniakarm" bzw. "ammoniakfrei" im Sinne der Erfindung beziehen sich dabei auf die dem Mittel zugesetzte Ammoniakmenge, wobei der Ammoniakzusatz sowohl als wässrige, alkoholische, wässrig-alkoholische oder anderweitige Lösung als auch durch Ammoniakgaseinleitung oder Zusatz von verflüssigtem Ammoniak erfolgen kann. Die Begriffe "ammoniakfrei" bzw. "ammoniakarm" im Sinne der Erfindung beziehen sich insbesondere nicht auf im erfindungsgemäßen Mittel freigesetzten Ammoniak, der durch Deprotonierung von als Salz zugesetzten Ammonium-Kationen, wie beispielsweise Ammoniumsulfat, entsteht. Bevorzugte, ammoniakarme Mittel enthalten weniger als 0,5 Gew.-%, insbesondere weniger als 0,1 Gew.-% und ganz besonders bevorzugt weniger als 0,05 Gew.-% zugesetzten Ammoniak, jeweils bezogen auf das Gesamtgewicht der Anwendungszubereitung. Ammoniakfrei im Sinne der Erfindung

sind solche Mittel, denen kein Ammoniak durch eine der oben beschriebenen Methoden zugesetzt wurde. Solche Mittel sind insbesondere erfindungsgemäß bevorzugt.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14,aromatischen Systeme, die mit einer quaternären Stickstoffgruppe substituiert sind, wie beispielsweise Basic Yellow 57, Basic Red 76, Basic Blue 99, Basic Brown 16 und Basic Brown 17. Die Verbindungen, die unter den Bezeichnungen Basic Yellow 87, Basic Orange 31 und Basic Red 51 bekannt sind, sind ganz besonders bevorzugte kationische direktziehende Farbstoffe. Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturnaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt. Die Gesamtmenge an direktziehenden Farbstoffen beträgt vorzugsweise höchstens 3 Gew.-%. Besonders gut als Vorstufen naturanaloger Haarfarbstoffe geeignet sind Derivate des 5,6-Dihydroxyindolins sowie physiologisch verträgliche Salze dieser Verbindungen mit einer organischen oder anorganischen Säure. Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin. Als Vorstufen naturanaloger Haarfarbstoffe hervorragend geeignet sind weiterhin Derivate des 5,6-Dihydroxyindols sowie physiologisch verträgliche Salze dieser Verbindungen mit einer organischen oder anorganischen Säure. Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

In einer weiteren, bevorzugten Ausführungsform der vorliegenden Erfindung enthält das Mittel weiterhin mindestens ein amphoteres Tensid. Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Erfindungsgemäß bevorzugte amphotere Tenside sind Verbindungen gemäß Formel (I) und/oder dessen physiologisch verträglichem Salze,

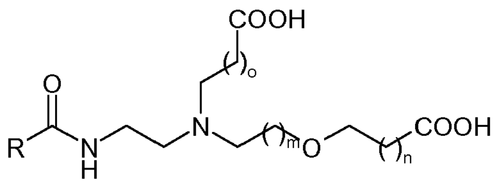

(I)

worin R für eine lineare oder verzweigte, gesättigte oder ungesättigte $C_{10}$-$C_{22}$-Alkylgruppe steht und m, n und o jeweils unabhängig voneinander für eine ganze Zahl 1, 2 oder 3 steht.

In einer bevorzugten Ausführungsform steht der Rest R gemäß Formel (I) für eine lineare $C_{12}$-$C_{18}$-Alkylgruppe. Besonders bevorzugte Verbindungen der Formel (I) werden unter der INCl-Bezeichnung Disodium Cocoamphodipropionate mit den Handelsnamen Miranol® C2M SF conc. (Rhodia), Amphoterge® K-2 (Lonza) und Monateric® CEM-38 (Unichema) vermarktet. Dabei leitet sich die Gruppe R-CO- gemäß Formel (I) von Fettsäuren des Kokosnussöls ab.

Der Zusatz im erfindungsgemäßen Mittel bewirkt eine hervorragende Avivage, insbesondere eine verbesserte Nasskämmbarkeit.

Die amphoteren Tenside sind in dem erfindungsgemäßen Mittel bevorzugt in einer Menge von 0,05 bis 30 Gew.-%, besonders bevorzugt von 0,1 bis 20 Gew.-%, jeweils bezogen auf das Gewicht des Mittels, enthalten.

In einer weiteren, bevorzugten Ausführungsform der vorliegenden Erfindung enthält das Mittel weiterhin mindestens ein kationisches oder amphoteres Polymer. ie kationischen oder amphoteren Polymere sind in dem erfindungsgemäßen Mittel bevorzugt in einer Menge von 0,01 bis 10 Gew.-%, besonders bevorzugt von 0,05 bis 5 Gew.-%, jeweils bezogen auf das Gesamtgewicht des anwendungsbereiten Mittels, enthalten.

Unter dem Begriff amphotere Polymere werden solche Polymere verstanden,

- die im Molekül sowohl freie Aminogruppen als auch freie -COOH- oder $SO_3H$-Gruppen enthalten und zur Ausbildung innerer Salze befähigt sind,
- zwitterionische Polymere, die im Molekül quartäre Ammoniumgruppen und -$COO^-$-Gruppen oder -$SO_3^-$-Gruppen enthalten, sowie
- Polymere, die -COOH-Gruppen oder $SO_3H$-Gruppen und quartäre Ammoniumgruppen enthalten.

Die oben genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer® erhältliche Acrylharz, das ein Copolymeres aus t-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Erfindungsgemäß bevorzugte amphotere Polymere sind solche Polymerisate, in denen sich eine kationische Gruppe ableitet von mindestens einem der folgenden Monomere:

(M1) Monomeren mit quartären Ammoniumgruppen der allgemeinen Formel (II),

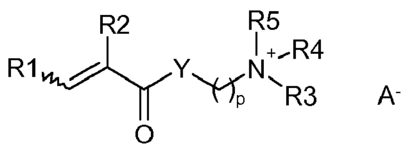

(II),

in der R1 und R2 unabhängig voneinander für Wasserstoff oder eine Methylgruppe stehen, R3, R4 und R5 unabhängig voneinander für Alkylgruppen mit 1 bis 4 Kohlenstoff-Atomen stehen, Y eine NH-Gruppe oder ein Sauerstoffatom, n eine ganze Zahl von 2 bis 5 und $A^-$ ein physiologisch verträgliches Anion einer organischen oder anorganischen Säure ist,

(M2) Monomeren mit quartären Ammoniumgruppen der allgemeinen Formel (III),

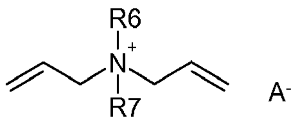
(III),

worin R6 und R7 unabhängig voneinander stehen für eine $C_1$-$C_4$-Alkylgruppe, insbesondere für eine Methylgruppe und

A- ein physiologisch verträgliches Anion einer organischen oder anorganischen Säure ist,

Wenn die kationische Gruppe der amphoteren Polymerisate sich vom Monomer des Typs (M1) ableiten, stehen in Formel (II) die Reste R3, R4 und R5 bevorzugt für Methylgruppen, Y ist bevorzugt eine NH-Gruppe und $A^-$ bedeutet bevorzugt ein Halogenid-, Methoxysulfat- oder Ethoxy-sulfat-Ion. Besonders bevorzugt ist es in diesem Falle Acrylamidopropyl-trimethyl-ammoniumchlorid als Monomer (M1) zu verwenden.
Wenn die kationische Gruppe der amphoteren Polymerisate sich vom Monomer des Typs (M2) ableiten, stehen in Formel (III) die Reste R6 und R7 bevorzugt für Methylgruppen und $A^-$ steht bevorzugt für ein Halogenidion, insbesondere für Chlorid oder Bromid.
Bevorzugte erfindungsgemäße amphotere Polymere sind Polymere, deren anionische Gruppe sich von mindestens einem Monomeren der Formel (IV) und/oder dessen physiologisch verträglichen Salzen ableitet,

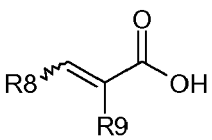
(IV),

in denen R8 und R9 unabhängig voneinander Wasserstoff oder Methylgruppen sind.
Als Monomeres gemäß Formel (IV) wird für die erfindungsgemäß bevorzugten amphoteren Polymerisate Acrylsäure verwendet.
Besonders bevorzugte Polymere sind Copolymere, die sich von mindestens einem Monomer (M1) bzw. (M2) mit dem Monomer gemäß Formel (IV) ableiten. Insbesondere sind Copolymere bevorzugt, die sich von den Monomeren (M2) und Monomeren gemäß Formel (IV) ableiten. Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 bzw. Merquat® 281 (Nalco) vertrieben.
Die amphoteren Polymere können sowohl direkt als auch in Salzform, die durch Neutralisation der Polymerisate, beispielsweise mit einem Alkalimetallhydroxid, erhalten wird, erfindungsgemäß eingesetzt werden.
Das erfindungsgemäß bevorzugte Mengenverhältnis des amphoteren Tensids mit der Formel (I) zum amphoteren oder kationischen Polymer beträgt bevorzugt 1 zu 5 bis 5 zu 1, besonders bevorzugt 1 zu 1 bis 1 zu 2.
Vorzugsweise stellen die anwendungsbereiten Färbemittel fließfähige Zubereitungen dar. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus kationischen, zwitterionischen, nichtionischen und, sofern benötigt, anionischen Tensiden und Emulgatoren ausgewählt sind. Anionische Tenside wurden vorstehend bereits erläutert, die übrigen Stoffe werden nachfolgend ausführlich beschrieben.
Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Weiterhin hat es sich als vorteilhaft erwiesen, wenn die erfindungsgemäßen Aufhellmittel nichtionogene grenzflächenaktive Stoffe enthalten. Nichtionische Tenside enthalten als hydrophile Gruppe z. B. eine Polyolgruppe, eine Polyalkylenglykolethergruppe oder eine Kombination aus Polyol- und Polyglykolethergruppe.

Als nichtionische Tenside eignen sich insbesondere $C_8$-$C_{22}$-Alkylmono- und -oligoglycoside und deren ethoxylierte Analoga. Insbesondere die nichtethoxylierten Verbindungen haben sich als besonders geeignet erwiesen. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die nichtionischen oder zwitterionischen Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Ebenfalls geeignet sind quaternäre Esterverbindungen, sogenannte "Esterquats". Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5,0 Gew.-% sind besonders bevorzugt.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Als Oxidationsmittel kommen Persulfate, Peroxodisulfate, Chlorite, Hypochlorite und insbesondere Wasserstoffperoxid oder dessen Anlagerungsprodukte an Harnstoff, Melamin sowie Natriumborat in Frage.

Bevorzugt wird als Oxidationsmittel Wasserstoffperoxid eingesetzt. Bevorzugt beträgt die Menge an Wasserstoffperoxid im anwendungsbereiten Mittel 0,5 bis 12 Gew.-%, bevorzugt 1 bis 8 Gew.-%, jeweils bezogen auf das anwendungsbereite Mittel.

Solche Oxidationsmittelzubereitungen sind vorzugsweise eine wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Die erfindungsgemäßen Mittel können zusätzlich auch Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. In diesen Fällen wird in der Regel eine Kombination aus Wasserstoffperoxid und Persulfaten bzw. Peroxodisulfaten eingesetzt, woraus eine Steigerung des Aufhellvermögens der Mittel resultiert. Daher kann es, sollte der Verbraucher den Wunsch nach einer sehr starken Blondierung verspüren, in einer weiteren Ausführungsform bevorzugt sein, wenn das erfindungsgemäße Färbemittel zusätzlich mindestens ein anorganisches Persulfatsalz bzw. Peroxodisulfatsalz in dem Mittel zum Aufhellen der keratinischen Fasern enthält. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein. Der Einsatz von Persulfatsalzen bzw. Peroxodisulfatsalzen erfolgt in der Regel in Form eines gegebenenfalls entstaubten Pulvers oder eines in Form gepressten Formkörpers.

Bei einer Anwendung von zusätzlichen Oxidationsmitteln wird das eigentliche Färbe- und/oder Aufhellmittel zweckmäßigerweise unmittelbar vor der Anwendung durch Mischung einer erfindungsgemäßen Zubereitung, enthaltend in einem kosmetischen Träger mindestens ein Oxidationsfarbstoffvorprodukt sowie als Gelbasis des Färbemittel 0,5 bis 10 Gew.-%, bezogen auf das Gesamtgewicht des anwendungsbereiten Mittels, einer nichtionischen Emulgatorkombination aus mindestens zwei ethoxylierten, linearen Fettalkoholen mit 8 bis 22 Kohlenstoffatomen, wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 35 bis 100 Mol Ethylenoxid besitzt, und mindestens ein verdickendes, polysaccharidisches Polymer enthält, sowie einer Zubereitung, enthaltend das zusätzliche Oxidationsmittel, insbesondere Wasserstoffperoxid, hergestellt.

Wird eine starke Aufhellung gewünscht, so ist es erfindungsgemäß bevorzugt, wenn zusätzlich eine Blondierzubereitung, enthaltend mindestens ein anorganisches Persulfatsalz bzw. Peroxodisulfatsalz, der Oxidationsmittelzubereitung vor Vermischung mit der erfindungsgemäßen Färbezubereitung beigemischt wird.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mindestens "zweizähnig" ist. Die Zahl der gebundenen Liganden hängt von der Koordinationszahl des zentralen Ions ab. Gebräuchliche und im Rahmen der vorliegenden Erfindung bevorzugte Chelatkomplexbildner sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Ferner können die erfindungsgemäßen Mittel weitere Wirk-, Hilfs- und Zusatzstoffe enthalten, wie beispielsweise nichtionische Polymere, kationische Polymere, zwitterionische und amphotere Polymere, anionische Polymere, Verdickungsmittel, Strukturanten wie Glucose, Maleinsäure und Milchsäure, haarkonditionierende Verbindungen wie Phospholipide sowie Silikonöle, Parfümöle, Dimethylisosorbid und Cyclodextrine, faserstrukturverbessernde Wirkstoffe, insbesondere Mono-, Di- und Oligosaccharide wie beispielsweise Glucose, Galactose, Fructose, Fruchtzucker und Lactose, Entschäumer wie Silikone, bevorzugt Dimethicon, Farbstoffe zum Anfärben des Mittels, Antischuppenwirkstoffe, Lichtschutzmittel, insbesondere Derivate von Benzophenon, Zimtsäure und Triazin, Konsistenzgeber wie Zuckerester, Polyolester oder Polyolalkylether, Fette und Wachse, Fettsäurealkanolamide, Quell- und Penetrationsstoffe, Trübungsmittel wie Latex, Styrol/PVP- und Styrol/Acrylamid-Copolymere, Pigmente, Stabilisierungsmittel für Wasserstoffperoxid und andere Oxidationsmittel, Treibmittel wie Propan-Butan-Gemische, $N_2O$, Dimethylether, $CO_2$ und Luft, und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen

der Kosmetika, 2. Auflage, Hüthig Buch Verlag, Heidelberg, 1989, verwiesen.

Ein zweiter Gegenstand der vorliegenden Erfindung ist ein Verfahren zum Färben menschlicher Haare, bei dem ein Mittel des ersten Erfindungsgegenstandes unmittelbar vor der Anwendung durch Vermischung des Färbemittels (a) und mit der Oxidationsmittelzubereitung (b) hergestellt wird, das nunmehr anwendungsbereite Mittel auf das Haar aufgetragen wird, für eine Einwirkzeit von 2 bis 20 Minuten, bevorzugt von 5 bis 15 Minuten auf dem Haar belassen wird, und anschließend das Haar ausgespült wird.

Die im Vergleich zu Handelsprodukten kurze Einwirkzeit im erfindungsgemäßen Verfahren garantiert bei gleichbleibenden Färbeergebnis ebenfalls eine verringerte Schädigung der Haare durch die verkürzte Kontaktzeit von schädigenden Substanzen mit dem Haar. Bezüglich weiterer bevorzugter Ausführungsformen der erfindungsgemäßen Verfahren gilt mutatis mutandis das zu den erfindungsgemäßen Mitteln Gesagte.

Die Anwendungstemperaturen beim erfindungsgemäßen Färbeverfahren können in einem Bereich zwischen 15 und 45 °C liegen. Nach der Einwirkungszeit wird das Haarfärbemittel durch Ausspülen, gegebenenfalls mit Hilfe eines Shampoos, von dem zu färbenden Haar entfernt. Das Nachwaschen mit einem Shampoo kann entfallen, wenn ein stark tensidhaltiger Träger, z. B. ein Färbeshampoo, verwendet wurde.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme bietet sich insbesondere dort an, wo Inkompatibilitäten der Inhaltsstoffe zu erwarten oder zu befürchten sind. Das anwendungsbereite Mittel wird bei solchen Systemen vom Verbraucher direkt vor der Anwendung durch Vermischen der Komponenten hergestellt. Ein Färbe- und/oder Aufhellungsmittel, bei dem die Oxidationsfarbstoffvorprodukte zunächst getrennt von der Oxidationsmittelzubereitung, enthaltend bevorzugt Wasserstoffperoxid, vorliegen, ist dabei bevorzugt.

Ein dritter Gegenstand der vorliegenden Erfindung ist daher eine Mehrkomponentenverpackungseinheit (Kit-of-Parts), enthaltend zwei getrennt konfektionierten Containern, wobei ein Container ein gelförmiges Färbemittel (a), enthaltend in einem kosmetischen Träger mindestens ein Oxidationsfarbstoffvorprodukt, und ein weiterer Container eine Oxidationsmittelzubereitung (b), enthaltend mindestens ein Oxidationsmittel, enthält, dadurch gekennzeichnet, dass die Gelbasis des Färbemittel (a) 0,5 bis 10 Gew.-%, bezogen auf das Gesamtgewicht des anwendungsbereiten Mittels, einer nichtionischen Emulgatorkombination aus mindestens zwei ethoxylierten, linearen Fettalkoholen mit 8 bis 22 Kohlenstoffatomen, wobei mindestens einer der ethoxylierten Fettalkohole einen mittleren, durchschnittlichen Ethoxylierungsgrad von 15 bis 35 besitzt und mindestens einer der ethoxylierten Fettalkohole einen hohen, durchschnittlichen Ethoxylierungsgrad von 40 bis 100 besitzt, und mindestens einen polymeren Verdicker enthält. Weiterhin enthält die Mehrkomponentenverpackungseinheit (Kit-of-Parts) zusätzlich eine Gebrauchsleitung. Darüber hinaus kann es bevorzugt sein, wenn weiterhin eine Applikationshilfe, wie beispielsweise ein Kamm oder ein Pinsel, und/oder eine persönliche Schutzausrüstung, wie beispielsweise Einweg-Handschuhe dem Kit beigefügt ist.

Wird eine besonders starke Aufhellwirkung durch Einsatz von Persulfatsalzen bzw. Peroxodisulfatsalzen gewünscht, ist es erfindungsgemäß bevorzugt, diese der erfindungsgemäßen Mehrkomponentenverpackungseinheit (Kit-of-Parts) in Form eines gegebenenfalls entstaubten Pulvers oder eines in Form gepressten Formkörpers als separat verpackte, zusätzliche Komponente beizufügen. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Ein weiterer Gegenstand der Erfindung ist die Verwendung der Mittel des ersten Erfindungsgegenstandes zum Färben menschlicher Haare bei verringerter Schädigung der Haare.

Ein weiterer Gegenstand der Erfindung ist die Verwendung der Mittel des ersten Erfindungsgegenstandes zur Verbesserung der Grauabdeckung bei verringerter Haarschädigung. Bezüglich weiterer bevorzugter Ausführungsformen der erfindungsgemäßen Verwendungen gilt mutatis mutandis das zu den erfindungsgemäßen Mitteln Gesagte.

Die nachfolgenden Beispiele sollen bevorzugte Ausführungsformen der Erfindung erläutern, ohne sie jedoch einzuschränken.

## Beispiele

### 1. Grauabdeckung bei erniedrigtem pH-Wert

#### 1.1. Herstellung der Färbecreme

Folgende Färbecremes mit gleicher Farbstoffmischung wurden hergestellt:

| | E1 | V1 | V2 |
|---|---|---|---|
| Eumulgin B1 | - | 0,6 | - |

(fortgesetzt)

| | E1 | V1 | V2 |
|---|---|---|---|
| Eumulgin B2 | 0,35 | 0,6 | 2,4 |
| Eumulgin B3 | 0,35 | - | - |
| Mergital CS 50 A | 1,40 | - | - |
| Xanthan | 0,14 | - | - |
| Hydrenol D | 7,0 | 6,6 | 9,6 |
| Lorol techn. | 4,2 | 2,4 | - |
| Lamesoft PO 65 | - | 2,0 | - |
| AkypoSoft 45 NV | - | 10,0 | - |
| Texapon K 14 S Special, 70% | - | 2,8 | - |
| Produkt W 37194 | - | 3,75 | - |
| Cutina GMS SE | - | - | 1,6 |
| Cutina AGS | - | - | 1,6 |
| Eutanol G | - | - | 1,6 |
| Texapon NSF, 27% | - | - | 3,52 |
| Phospholipid EFA | - | - | 0,1 |
| Lanette E, Pulver | - | - | 0,56 |
| Kalium Oleat, 12,5% | - | - | 2,4 |
| Ammonium Carbomer, 1% | - | - | 12,0 |
| 1,2-Propandiol | 0,7 | - | |
| $Na_4$-EDTA, Pulver, 87% | - | - | 0,2 |
| Monoethanolamin | 5,5 | 4,5 | 6,0 |
| Farbstoffmischung* | 3,37 | 3,37 | 3,37 |
| L-Serin | - | - | 0,5 |
| Arginin | 1,0 | 1,0 | 1,0 |
| Ammoniumsulfat, techn. rein | 2,0 | 2,0 | - |
| Ascorbinsäure | 0,2 | 0,2 | 0,2 |
| Natriumsulfit, wasserfrei, 96% | 0,2 | 0,2 | 0,2 |
| HEDP, 60% | 0,2 | 0,2 | 0,2 |
| Natriumsilikat 40 / 42 | 0,5 | 0,5 | 0,5 |
| Wasser | ad 100 | ad 100 | ad 100 |

*Farbstoffmischung, enthaltend 53,4 Gew.-% p-Toluylendiaminsulfat, 7,4 Gew.-% 4-Amino-3-methylphenol, 1,2 Gew.-% 1,5-Dihydroxynaphthalin, 11,9 Gew.-% Resorcin, 12,8 Gew.-% 4-Chlorresorcin, 6,5-Gew.-% 5-Amino-2-methylphenol, 4,5 Gew.-% 2-Amino-6-chloro-4-nitrophenol und 2,4 Gew.-% 3-Amino-2-methylamin-6-methoxypyridin, jeweils bezogen auf das Gesamtgewicht der Farbstoffmischung.

Hydrenol® D — $C_{16}$-$C_{18}$-Fettalkohol (INCI-Bezeichnung: Cetearyl alcohol) (Cognis)
Lorol® tech. — $C_{12}$-$C_{18}$-Fettalkohol (INCI-Bezeichnung: Coconut alcohol) (Cognis)

| | |
|---|---|
| Eumulgin® B1 | $C_{16}$-$C_{18}$-Fettalkohol, ethoxyliert (12 EO) (INCI-Bezeichnung: Ceteareth-12) (Cognis) |
| Eumulgin® B2 | $C_{16}$-$C_{18}$-Fettalkohol, ethoxyliert (20 EO) (INCI-Bezeichnung: Ceteareth-20) (Cognis) |
| Eumulgin® B3 | C16-C18-Fettalkohol, ethoxyliert (30 EO) (INCI-Bezeichnung: Ceteareth-20) (Cognis) |
| Mergital® CS 50 | C16-C18-Fettalkohol, ethoxyliert (50 EO) (INCI-Bezeichnung: Ceteareth-50) (Cognis) |
| Lamesoft® PO 65 | C12-C18-Fettalkohol-Polyglucosidether, Glycerinmonooleat (INCI-Bezeichnung: Coco-Glucoside, Glyceryl Oleate, aqua) (Cognis) |
| AkypoSoft® 45 NV | C12-C14-Fettalkoholacetat, ethoxyliert (4,5 EO), Natrium-Salz (INCI-Bezeichnung: Sodium Laureth-5 Carboxylate) (KAO) |
| Texapon® K 14 S Special | C14-Fettalkoholsulfat, ethoxyliert (3 EO) Natrium-Salz (INCI-Bezeichnung: Sodium Myreth Sulfate) (Cognis) |
| Produkt W 37194 | Natrium acrylat/Trimethylammoniopropylacrylamid chlorid copolymer (INCI-Bezeichnung: Acrylamidopropyltrimonium chloride/Acrylates Copolymer) (Stockhausen) |
| Cutina® GMS SE | Stearinsäureglycerylester (INCI-Bezeichnung: Glyceryl Stearate) (Cognis) |
| Cutina® AGS | Ethylenglycoldistearylester (INCI-Bezeichnung: Glycol Distearate) (Cognis) |
| Eutanol® G | 2-Octyldodecanol (INCI-Bezeichnung: Octyldodecanol) (Cognis) |
| Texapon® NSF, 27% | C12-Fettalkoholsulfat, ethoxyliert (2 EO) Natrium-Salz (INCI-Bezeichnung: Sodium Laureth Sulfate) (Cognis) |
| Phospholipid EFA | zwitterionisches Phospholid (INCI-Bezeichnung: Linoleamidopropyl PG-dimonium chloridephosphate) (Uniqema) |
| Lanette® E, Pulver | C16-C18-Fettalkoholsulfat, Natrium-Salz (INCI-Bezeichnung: Sodium Cetearyl Sulfate) (Cognis) |
| Natriumsilikat 40/42 | Natronwasserglas |

Die Fettbasis wurde jeweils zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

Bei den Rezepturen V1 bis V2 handelt es sich um nicht erfindungsgemäße Vergleichsrezepturen, welche neben der fehlenden, erfindungsgemäßen Gelbasis außerdem signifikante Anteile an anionischen Tensiden enthalten (V1 mit AkypoSoft und Texapon K14S: 12,0 Gew.-%, V2 mit Texapon NSF, Kaliumoleat und Lanette E: 1,75 Gew.-%). Die Rezeptur E1 ist als ein erfindungsgemäßes Beispiel frei von anionischen Tensiden.

1.2. Vermischen mit der Entwicklerdispersion und Applikation

Jede Färbecreme wurde im Gewichtsverhältnis 1:1 mit einer wie folgt zusammengesetzten Entwicklerdispersion ausgemischt.

| Rohstoff | Gew.-% |
|---|---|
| Natronlauge, 45 Gew.-%, techn. | 0,82 |
| Dipicolinsäure | 0,10 |
| Dinatriumpyrophosphat | 0,03 |
| HEDP, 60% | 1,50 |
| Texapon NSO | 2,00 |
| Dow Corning DB 110 A | 0,07 |
| Aculyn 33A | 4,50 |
| Wasserstoffperoxid 50 % | 10,00 |
| Wasser | ad 100 |

| | |
|---|---|
| Texapon® NSO | Laurylethersulfat, Natriumsalz (ca. 27,5% Aktivsubstanz; INCI-Bezeichnung: Sodium Laureth Sulfate) (Cognis) |
| Aculyn® 33 | Acrylpolymer (ca. 28% Festkörper in Wasser; INCI-Bezeichnung: Acrylates Copolymer) (Rohm & Haas) |
| Dow Corning® DB 110 A | nicht ionische Silikonemulsion (INCI-Bezeichnung: Dimethicon) (Dow Corning) |

Die Menge des Alkalisierungsmittels in der Färbecreme wurde so gewählt, dass anwendungsbereiten Färbemischungen jeweils einen pH-Wert von 9,5 besaßen (E1-a, V1-a, V2-a).

Außerdem wurden analoge, anwendungsbereite Färbemischungen jeweils mit einem pH-Wert von 8,5 durch Reduktion der Alkalisierungsmittelmenge hergestellt (E1-b, V1-b, V2-b).

Für den Färbung wurde auf Strähnen von weißem Büffelbauchhaar bzw. hellbraunen Haares (Code: Kerling 6-0) von ca. 0,7 g Gewicht die 4-fache Menge der fertigen Anwendungsmischungen (E1-a, V1-a, V2-a sowie E1-b, V1-b, V2-b) appliziert. Nachdem die Strähnen für 10 Minuten bei 32 °C gefärbt wurden, wurden sie mit einem handelsüblichen Shampoo gewaschen und mit einem Föhn getrocknet.

1.3. Auswertung der Grauabdeckung

Für die Berechnung des Farbabstands zwischen ungefärbten und gefärbten Haaren wurden die gefärbten und ungefärbten Strähnen mit Hilfe des Farbmessgerät der Firma Datacolor, Typ Spectraflash 450 vermessen. Der Farbabstand ΔE berechnet sich nach folgender Formel:

$$\Delta E_{1;2} = \sqrt{(L_1 - L_2)^2 + (a_1 - a_2)^2 + (b_1 - b_2)^2}$$

Hierbei steht der L-Wert für die Helligkeit (je geringer der L-Wert ist, desto größer ist die Farbintensität), während der a-Wert ein Maß für den Rotanteil einer Farbe ist (d. h. je größer der a-Wert ist, umso größer ist der Rotanteil). Der b-Wert ist ein Maß für den Blauanteil der Farbe, wobei der Blauanteil umso größer ist, je negativer der b-Wert ist.

Zur Berechnung der Grauabdeckung wurde der Farbabstand zwischen gefärbtem, weißen Haar (BB) und gefärbtem, hellbraunen Haar (NH) in ein Verhältnis zum Farbabstand zwischen ungefärbtem, weißen Haar (NS) und gefärbtem, hellbraunen Haar (NH) gemäß folgender Formel (Grauabdeckungs-Index GAI) gesetzt.

$$GAI = \left(1 - \frac{\Delta E_{BB;NH}}{\Delta E_{NS;NH}}\right) \cdot 100$$

Mit gemessenen ΔE-Werten ergeben sich für die oben beschriebenen Färbungen folgende Graudeckungs-Indices (Tabelle 1).

Tabelle 1.

| Färbemischung | **E1-a** (pH 9,5) | **E1-b** (pH 8,5) | **V1-a** (pH 9,5) | **V1-b** (pH 8,5) | **V2-a** (pH 9,5) | **V2-b** (pH 8,5) |
|---|---|---|---|---|---|---|
| Farbabstand $\Delta E_{BB;NH}$ | 7,04 | 7,61 | 6,31 | 8,69 | 6,72 | 8,99 |
| Farbabstand $\Delta E_{NS;NH}$ | 54,16 | 54,61 | 53,71 | 55,25 | 54,31 | 55,72 |
| Grauabdeckungs-Index **GAI** | **87,0** | **86,1** | **88,2** | **84,3** | **87,6** | **83,9** |
| $\Delta GAI_{pH9,5;pH8,5}$ | **0,9** | | **3,9** | | **3,7** | |

Aus Tabelle 1 ist eindeutig ersichtlich, dass die erfindungsgemäße Coloration E1 auch bei einer signifikanten Verringerung des pH-Werts eine nahezu gleichbleibende Grauabdeckung erzielt, während die handelsüblichen Färbungen V1 und V2 deutliche Einbußen in ihrem Grauabdeckungsvermögen bei einer Erniedrigung des pH-Werts zeigen.

**2. Verminderung der Haarschädigung durch Erniedrigung des pH-Werts**

2.1. Methodenbeschreibung zur NIR- Analytik von behandeltem Haar

<u>Qualitative Bestimmung der Haarstrukturschädigung mittels Clusteranalyse:</u>

Haare können durch oxidative Behandlung wie Färben oder Dauerwellen geschädigt werden. Cystin ist ein

Bestandteil des menschlichen Haares. Cystin zeigt bei einer Wellenlänge von 6200 $cm^{-1}$ - 5500 $cm^{-1}$ typische Schwingungen im NIR-Spektrum (Nah-Infra-Rot). Die in Cystin enthaltene Disulfid-Brücke wird bei einer Haarbehandlung oxidativ gebrochen, und der resultierende Thiol durch weitere Oxidation in die Sulfonsäure-Gruppe der Cysteinsäure überführt. Gleichzeitig verändert sich das Haar also hinsichtlich des Cysteinsäuregehaltes, welcher mit stärkerer, oxidativer Schädigung zunimmt. Cysteinsäure zeigt sich im NIR-Spektrum bei einer Wellenlänge von 5020 $cm^{-1}$ - 4020 $cm^{-1}$. Die Abnahme an Cystin und die Zunahme an Cysteinsäure stellt daher einen Indikator für die Schädigung des Haares dar.

Die Aufnahme der Spektren wird mit einem MPATM FT-NIR-Spektrometer der Bruker Optik GmbH (Ettlingen, BRD) durchgeführt. Der Nah-Infrarot (NIR)-Bereich umfasst den Wellenzahlbereich von 12500 $cm^{-1}$ bis 4000 $cm^{-1}$ und ist für Oberton- und Kombinationsschwingungen von z.B. CH-, OH- und NH-Gruppen charakteristisch. Die Messung der Proben erfolgt mit dem Integrationskugelmodul an sechs verschiedenen Probenpositionen in diffuser Reflexion. Für die Analyse der gemessenen NIR-Spektren wird der Wellenzahlbereich 8000 $cm^{-1}$ - 4020 $cm^{-1}$ gewählt. Für die chemometrische Auswertung wird das geräteunabhängige Softwarepaket Unscrambler (Fa. CAMO, Oslo, Norwegen) verwendet.

Anhand einer PCA (Principal Compound Analysis) kann man die Separation der Spektren in sogenannte "Cluster" erkennen Je größer die Abstände der Cluster zu dem der unbehandelten Muster sind, desto größer ist die spektrale Abweichung einhergehend mit dem Ausmaß der Haarstrukturveränderung. Auf diese Art und Weise lassen sich die Haare nach Behandlung mit Aufhellern und anschließender Vermessung im NIR in Cluster einteilen. Die Stärke der Veränderung des behandelten Haares ist sofort erkennbar.

Quantitative Bestimmung der Haarstrukturschädigung mittels Kalibrationserstellung:

NIR-Spektren lassen sich auch quantitativ auswerten, wenn Referenzwerte vorhanden sind. Bei der Modellbildung wird mit Hilfe eines Partial Least Squares (PLS)-Algorithmus eine Korrelation zwischen spektroskopischen Daten und den entsprechenden Konzentrationswerten der einzelnen Komponenten berechnet. Referenzwerte erhält man, indem man Proben im Labor über ein anderes unabhängiges analytisches Verfahren quantitativ bestimmt. Das PLS-Modell wird durch Veränderung des Frequenzbereiches, mathematische Datenvorbehandlung, Auswahl der geeigneten Faktorenzahl und automatischer Ausreißererkennung verbessert.

Eine detaillierte Beschreibung der Methode findet sich in Evaluation of Physical Properties of Human Hair by Diffuse Reflectance Near-Infrared Spectroscopy, Y. Miyamae, Y. Yamakawa und Y. Ozaki in Applied Spectroscopy, 2007, Vol. 61 (2), S. 212 ff.

2.2 Färbungen

Folgende Färbecremes wurden nach dem unter 1.1 beschriebenen Verfahren für jeweils 2 unterschiedliche pH-Werte (nach Mischung mit der Entwicklerdispersion) hergestellt.

| | **E2-a** | **E2-b** | **E3-a** | **E3-b** | **E4-a** | **E4-b** | **E5-a** | **E5-b** | **E6-a** | **E6-b** |
|---|---|---|---|---|---|---|---|---|---|---|
| Creme[1] | 25,2 | 25,2 | 25,2 | 25,2 | 25,2 | 25,2 | 25,2 | 25,2 | 25,2 | 25,2 |
| p-Toluylendiaminsulfat | 1,41 | 1,41 | 1,30 | 1,30 | 0,99 | 0,99 | 0,54 | 0,54 | 2,72 | 2,72 |
| 4-Amino-3-methyl-phenol | - | - | 0,38 | 0,38 | - | - | - | - | 0,37 | 0,37 |
| 2,6-Dihydroxy-3,4-dimethylpyridin | - | - | - | - | - | - | 0,18 | 0,18 | - | - |
| 1,5-Dihydroxynaphthalin | 0,05 | 0,05 | - | - | 0,05 | 0,05 | - | - | 0,05 | 0,05 |
| Resorcin | 0,40 | 0,40 | 0,68 | 0,68 | 0,07 | 0,07 | - | - | 0,61 | 0,61 |
| 3-Aminophenol | - | - | 0,05 | 0,05 | - | - | - | - | - | - |
| 5-Amino-2-methyl-phenol | - | - | 0,15 | 0,15 | - | - | - | - | 0,35 | 0,35 |
| 4-Chlorresorcin | 0,43 | 0,43 | - | - | 0,11 | 0,11 | - | - | 0,65 | 0,65 |
| 2-Methylresorcin | - | - | - | - | 0,34 | 0,34 | 0,15 | 0,15 | - | - |

(fortgesetzt)

| | E2-a | E2-b | E3-a | E3-b | E4-a | E4-b | E5-a | E5-b | E6-a | E6-b |
|---|---|---|---|---|---|---|---|---|---|---|
| 2-Amino-6-chloro-4-nitrophenol | 0,05 | 0,05 | 0,07 | 0,07 | 0,05 | 0,05 | 0,02 | 0,02 | 0,05 | 0,05 |
| 3-Amino-2-methylamino-6-methoxypyridin | 0,08 | 0,08 | - | - | 0,06 | 0,06 | - | - | 0,10 | 0,10 |
| 1,2-Propandiol | - | - | 0,45 | 0,45 | - | - | - | - | 1,1 | 1,1 |
| Arginin | 1,0 | 1,0 | 1,0 | 1,0 | 1,0 | 1,0 | 1,0 | 1,0 | 1,0 | 1,0 |
| Ammoniumsulfat | 2,0 | 2,0 | 2,0 | 2,0 | 2,0 | 2,0 | 2,0 | 2,0 | 2,0 | 2,0 |
| Ascorbinsäure | 0,2 | 0,2 | 0,2 | 0,2 | 0,2 | 0,2 | 0,2 | 0,2 | 0,2 | 0,2 |
| Natriumsulfit, wasserfrei, 96% | 0,2 | 0,2 | 0,2 | 0,2 | 0,2 | 0,2 | 0,2 | 0,2 | 0,2 | 0,2 |
| HEDP, 60% | 0,2 | 0,2 | 0,2 | 0,2 | 0,2 | 0,2 | 0,2 | 0,2 | 0,2 | 0,2 |
| Natriumsilikat 40 / 42 | 0,5 | 0,5 | 0,5 | 0,5 | 0,5 | 0,5 | 0,5 | 0,5 | 0,5 | 0,5 |
| **Monoethanolamin**[2] | ad mpH **9,5** | ad mpH **8,5** | ad mpH **9,6** | ad mpH **8,5** | ad mpH **9,6** | ad mpH **8,5** | ad mpH **9,8** | ad mpH **8,5** | ad mpH **9,5** | ad mpH **8,5** |
| Wasser | ad 100 | ad 100 | ad 100 | ad 100 | ad 100 | ad 100 | ad 100 | ad 100 | ad 100 | ad 100 |

[1]Creme, bestehend aus 2,0 Gew.-% Eumulgin B2, 2,0 Gew.-% Eumulgin B3, 7,9 Gew.-% Mergital CS 50 A, 0,8 Gew.-% Xanthan, 39,7 Gew.-% Hydrenol D, 23,8 Gew.-% Lorol techn., 4,0 Gew.-% 1,2-Propandiol, 11,9 Gew.-% Merquat 281 und 7,9 Gew.-% Amphoterge K-2, jeweils bezogen auf die Gesamtmenge der Creme.
[2]Die Monoethanolamin-Menge wurde so gewählt, dass der angegebene Misch-pH-Wert (mpH) der anwendungsbereiten Färbemischung nach Vermischung mit der unter 1.2 beschriebenen Entwicklerdispersion erreicht wurde.

Merquat® 281 Dimethyldiallylammoniumchlorid/Acrylsäure Copolymer (INCI-Bezeichnung: Polyquaternium-22) (Nalco)
Amphoterge® K-2 (INCI-Bezeichnung: Disodium Cocoamphodipropionate) (Lonza)

Die Ausfärbungen erfolgten analog zu 1.2 auf hellbraunem Haar (Kerling 6/0) nach Vermischen der Färbecreme mit der unter 1.2 beschriebenen Entwicklerdispersion (1:1 nach Gewicht). Die Färbeergebnisse der Färbung bei pH ca. 9,5 unterschieden sich nicht von denen bei pH 8,5. In jedem Fall wurden intensive und dauerhafte Färbungen erzielt.

2.3 Ergebnisse der Cysteinsäurebestimmung

Nach Mittelwertbildung aus jeweils 9 unabhängigen Messungen mit unter 2.1 beschriebenen Methode wurden für die unter 2.2 beschriebenen Färbungen die jeweilige der Cysteinsäure-Menge und damit der Schädigungsgrad der Strähnen ermittelt (Tabelle 2).

Tabelle 2.

| Färbemischung | NIR-Wert [mol/100 mol Aminosäure] | △ der NIR-Werte einer Nuance bei pH-Erniedrigung |
|---|---|---|
| Kerling 6/0, unbehandelt | 0,7 | |
| **E2-a** (pH 9,5) | 1,7 | 0,3 |
| **E2-b** (pH 8,5) | 1,4 | |

(fortgesetzt)

| Färbemischung | NIR-Wert [mol/100 mol Aminosäure] | △ der NIR-Werte einer Nuance bei pH-Erniedrigung |
|---|---|---|
| **E3-a** (pH 9,6) | 1,7 | 0,3 |
| **E3-b** (pH 8,5) | 1,4 | |
| **E4-a** (pH 9,6) | 1,5 | 0,3 |
| **E4-b** (pH 8,5) | 1,2 | |
| **E5-a** (pH 9,8) | 1,4 | 0,3 |
| **E5-b** (pH 8,5) | 1,1 | |
| **E6-a** (pH 9,5) | 1,5 | 0,2 |
| **E6-b** (pH 8,5) | 1,3 | |

Aus Tabelle 2 kann ersehen werden, dass die Färbungen mit Erniedrigung des pH-Werts einen deutlich geringeren Cysteinsäureanteil in der Strähne bewirken. Damit wird eine Verringerung der Schädigung der Haare durch den oxidativen Färbevorgang bei gleichbleibendem Färbeergebnis erzielt.

**Patentansprüche**

1. Mittel zum Färben keratinischer Fasern, insbesondere menschlicher Haare, bestehend aus mindestens zwei getrennt konfektionierten Containern, wobei ein Container ein gelförmiges Färbemittel (a), enthaltend in einem kosmetischen Träger mindestens ein Oxidationsfarbstoffvorprodukt, und ein weiterer Container eine Oxidationsmittelzubereitung (b), enthaltend mindestens ein Oxidationsmittel, enthält, **dadurch gekennzeichnet, dass**

    i. die Gelbasis des Färbemittel (a)

        0,5 bis 10 Gew.-%, bezogen auf das Gesamtgewicht des anwendungsbereiten Mittels, einer nichtionischen Emulgatorkombination aus mindestens zwei ethoxylierten, linearen Fettalkoholen mit 8 bis 22 Kohlenstoffatomen,
        wobei mindestens einer der ethoxylierten Fettalkohole einen mittleren, durchschnittlichen Ethoxylierungsgrad von 15 bis 35 Mol Ethylenoxid besitzt und mindestens einer der ethoxylierten Fettalkohole einen hohen, durchschnittlichen Ethoxylierungsgrad von 40 bis 100 Mol Ethylenoxid besitzt,
        und mindestens einen polymeren Verdicker enthält,

    ii. das anwendungsbereite Mittel einen pH-Wert von 7 bis 8,9 besitzt, und
    iii. das anwendungsbereite Mittel anionische Tenside in einem Gesamtgehalt von weniger als 0,3 Gew.-%, bezogen auf das Gesamtgewicht der Anwendungszubereitung, enthält.

2. Mittel nach Anspruch 1, **dadurch gekennzeichnet, dass** der ethoxylierte, lineare Fettalkohol mit einen hohen, durchschnittlichen Ethoxylierungsgrad des Färbemittels (a) einen durchschnittlichen Ethoxylierungsgrad von 40 bis 60, bevorzugt 45 bis 55 besitzt.

3. Mittel nach Anspruch 1 oder 2, **dadurch gekennzeichnet, dass** das Gewichtsverhältnis der Menge der ethoxylierten Fettalkohole mit einem mittleren, durchschnittlichen Ethoxylierungsgrad zu der Menge der ethoxylierten Fettalkohole mit einem hohen, durchschnittlichen Ethoxylierungsgrad im Färbemittel (a) einen Wert von 3:1 bis 1:5, bevorzugt von 2:1 bis 1:3, besitzt.

4. Mittel nach einem der Ansprüche 1 bis 3, **dadurch gekennzeichnet, dass** polymere Verdicker im Färbemittel (a) zu 0,005 bis 1,0 Gew.-%, bezogen auf das Gesamtgewicht der Anwendungszubereitung, enthalten ist.

5. Mittel nach einem der Ansprüche 1 bis 4, **dadurch gekennzeichnet, dass** der polymere Verdicker des Färbemittels (a) ein verdickendes, polysaccharidisches Polymer ist.

6. Mittel nach einem der Ansprüche 1 bis 5, **dadurch gekennzeichnet, dass** der polymere Verdicker des Färbemittels (a) Xanthan ist.

7. Mittel nach einem der Ansprüche 1 bis 6, **dadurch gekennzeichnet, dass** das Färbemittel (a) frei von anionischen Tensiden ist.

8. Mittel nach einem der Ansprüche 1 bis 7, **dadurch gekennzeichnet, dass** das Färbemittel (a) eine Kombination von mindestens zwei Alkalisierungsmitteln enthält,
wobei mindestens ein Alkalisierungsmittel ein Alkanolamin ist, ausgewählt aus Monoethanolamin, Monoisopropanolamin, 2-Amino-2-methylpropanol, 2-Amino-2-methyl-1,3-propandiol, 2-Amino-2-ethyl-1,3-propandiol, 2-Amino-2-methylbutanol, 2-Aminobutanol, N,N-Dimethyl-ethanolamin, Methylglucamin, Triethanolamin, Diethanolamin und Triisopropanolamin,
und wobei mindestens ein Alkalisierungsmittel eine basische Aminosäure ist.

9. Mittel nach Anspruch 8, **dadurch gekennzeichnet, dass** das Alkanolamin Monoethanolamin ist.

10. Mittel nach einem der Ansprüche 7 bis 8, **dadurch gekennzeichnet, dass** die basische Aminosäure ausgewählt ist aus der Gruppe, die gebildet wird aus Lysin, Guanidin, Ornithin und Arginin.

11. Mittel nach einem der Ansprüche 1 bis 10, **dadurch gekennzeichnet, dass** das Färbemittel (a) ammoniakarm, bevorzugt ammoniakfrei, ist.

12. Mittel nach einem der Ansprüche 1 bis 11, **dadurch gekennzeichnet, dass** das Mittel zusätzlich mindestens ein direktziehenden Farbstoff enthält.

13. Verfahren zum Färben menschlicher Haare, bei dem ein Mittel nach einem der Ansprüche 1 bis 12 unmittelbar vor der Anwendung durch Vermischung des Färbemitttels (a) und mit der Oxidationsmittelzubereitung (b) hergestellt wird, das nunmehr anwendungsbereite Mittel auf das Haar aufgetragen wird, für eine Einwirkzeit von 2 bis 20 Minuten, bevorzugt von 5 bis 15 Minuten, auf dem Haar belassen wird, und anschließend das Haar ausgespült wird.

14. Verwendung eines Mittels gemäß einem der Ansprüche 1 bis 12 zum Färben menschlicher Haare bei verringerter Haarschädigung.

15. Verwendung eines Mittels gemäß einem der Ansprüche 1 bis 12 zur Verbesserung der Grauabdeckung bei verringerter Haarschädigung.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Y. Miyamae ; Y. Yamakawa ; Y. Ozaki.** Evaluation of Physical Properties of Human Hair by Diffuse Reflectance Near-Infrared Spectroscopy. *Applied Spectroscopy,* 2007, vol. 61 (2), 212 ff